(19) **Europäisches Patentamt**
**European Patent Office**
**Office européen des brevets**

(11) **EP 4 324 471 A1**

(12) **EUROPEAN PATENT APPLICATION**

(43) Date of publication:
**21.02.2024 Bulletin 2024/08**

(21) Application number: **22190349.5**

(22) Date of filing: **15.08.2022**

(51) International Patent Classification (IPC):
*A61K 36/708* (2006.01)    *A61K 36/886* (2006.01)
*A61K 36/258* (2006.01)    *A61Q 11/00* (2006.01)
*A61P 1/02* (2006.01)

(52) Cooperative Patent Classification (CPC):
(C-Sets available)
**A61P 1/02; A61K 36/258; A61K 36/708;
A61K 36/886; A61Q 11/00**                    (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universitätsmedizin der
Johannes Gutenberg-Universität Mainz
55131 Mainz (DE)**

(72) Inventors:
- **MÜLLER-HEUPT, Lena Katharina
  64572 Büttelborn (DE)**
- **WIESMANN, Nadine
  55129 Mainz (DE)**
- **SCHRÖDER, Sofia Franziska
  55131 Mainz (DE)**

(74) Representative: **Patentanwälte Dr. Keller,
Schwertfeger
Partnerschaft mbB
Westring 17
76829 Landau (DE)**

(54) **COMPOSITIONS FOR USE IN THE PREVENTION OR TREATMENT OF ORAL OR DENTAL DISORDERS OR DYSFUNCTIONS**

(57)    The invention relates to a composition comprising rhubarb *(Rheum palmatum)* root extract and one or more additives selected from the group consisting of Acemannan and/or Glucomannan, or plant extracts containing said compounds for use in the prevention or treatment of oral or dental disorders or dysfunctions. Furthermore, the invention relates to a mouth rinse solution or dentifrice composition for use in oral or dental care.

**EP 4 324 471 A1**

(52) Cooperative Patent Classification (CPC): (Cont.)

C-Sets
**A61K 36/258, A61K 2300/00;**
**A61K 36/708, A61K 2300/00;**
**A61K 36/886, A61K 2300/00**

**Description**

**Field of the Invention**

The invention relates to compositions comprising rhubarb *(Rheum palmatum)* root extract and one or more additives selected from the group consisting of Acemannan and/or Glucomannan, or plant extracts containing said compounds for use in the prevention or treatment of oral or dental disorders or dysfunctions. The invention furthermore relates to mouth rinse solutions or dentifrice composition for use in oral or dental care, comprising a composition according to the present invention.

**Background of the Invention**

The oral cavity is an anatomical space that includes many important structures such as lips, oral vestibule, palate, and teeth.

The oral cavity offers excellent living conditions for microorganisms due to its constant temperature and high humidity. The oral microbiome comprises a variety of bacteria, yeasts, and sometimes even protozoa, which are in an ecological balance with each other. This balance is important for oral health. Many of the inflammatory diseases like caries, gingivitis, and periodontitis are not caused by new pathogens introduced from the outside, but rather by a shift in the balance towards a predominance of certain bacterial species always present in the oral cavity, resulting in an opportunistic infection.

More than 300 different species of bacteria have been identified in the oral cavity, and taking into account the archaea, about 600 different species of prokaryotes can occur in the oral cavity. The initial contamination with germs already takes place during birth in the birth canal. The acid-forming streptococci (*Streptococcus mutans*), which are partly responsible for caries, can already be observed after the eruption of the first teeth.

The most common diseases that occur in the oral cavity include dental caries and inflammation of the gums (gingivitis), periodontium (periodontitis, periodontosis), and mucous membrane (mucositis, stomatitis, peri-implantitis). Without treatment, these diseases can result in tooth or implant loss. Inflammations of the tongue (glossitis) and other pathological changes of the tongue are also included. Less frequently, tumors, cysts, leukoplakias, and fungal infections also occur in the oral cavity.

Compositions that exhibit antimicrobial and anti-inflammatory properties are known ("What we already know about rhubarb: a comprehensive review", Chinese Medicine. 2020, Vol 15: 88). Many of them are based on plant extracts, such as extracts derived from rhubarb root, and are part of traditional Chinese medicine, such as the compositions disclosed in CN1263484C, CN107595953A, or CN101491614A.

The randomized controlled trial "The curative effect of rhubarb extract on severe periodontitis in patients with diabetes mellitus" (2018 Dec;27(6):633-636) reveals the use of rhubarb extract after scaling and root planing therapy for the treatment of periodontitis of diabetes mellitus patients with good clinical effectiveness. Rhubarb is also known to be effective in wound healing ("Rheum emodi L. (rhubarb) promotes wound healing by decreasing inflammatory markers and enhancing accumulation of biomolecules", Annals of Phytomedicine 6(2): 114-118, 2017).

JPS5758612A discloses oral compositions suitable for prevention of dental caries and periodontal diseases and screened various plants as natural substance additives. As a result, rhubarb has been detected to become a promising candidate. Compositions comprising one or two or more organic solvent extracts of plants selected from Yoba-Hei, Rhubarb, Red-faced Red-eye-bean and *Phellinus cinerea* are specifically disclosed.

For use in other medical indications, compositions are known that contain rhubarb root extract and *Aloe vera* extract. *Aloe vera* gel exhibits anti-microbial properties at higher concentrations and has been proven to be suitable for oral health care ("Antibacterial Effect of Aloe vera Gel against Oral Pathogens: An In-vitro Study", Journal of Clinical and Diagnostic Research, 2016, Vol-10(11): ZC41-ZC44).

CN103479817A discloses a wound healing gel that contains rhubarb root extract and *Aloe vera* extract, among a host of other ingredients. CN108420933A discloses a composition for treating burns containing rhubarb and *Aloe vera* beside several other ingredients.

There have been numerous clinical trials using *Aloe vera* to treat a wide variety of wound injuries ("The Effect of Aloe vera Clinical Trials on Prevention and Healing of Skin Wound: A Systematic Review", IJMS Vol 44, No 1, 2019).

In addition, the use of an *Aloe vera* gel in combination with scaling and root planing therapy for the treatment of periodontitis has been revealed ("Clinical evaluation of effects of local application of Aloe vera gel as an adjunct to scaling and root planing in patients with chronic periodontitis", J Dent Shiraz Univ Med Sci, 2017 Sep; 18(3): 165-172).

The product "Pyralvex®" of the company MEDA Pharma GmbH & Co. KG relates to a solution for the treatment of inflammations of the oral mucosa (stomatitis) and gingivitis, which contains rhubarb root extract and salicylic acid as the main active ingredients.

It is desirable to further increase the effectiveness and efficiency of the known compositions.

## Description of the Invention

Against this background, it is the object of the present invention to provide alternative compositions which can enhance wound healing and exhibit an anti-bacterial effect suitable for the use in the prevention or treatment of oral or dental disorders or dysfunctions.

This object is solved by the subject-matter of claim 1, in particular by a composition comprising rhubarb *(Rheum palmatum)* root extract and one or more additives selected from the group consisting of Acemannan and/or Glucomannan, or plant extracts containing said compounds for use in the prevention or treatment of oral or dental disorders or dysfunctions.

Acemannan, a synonym for Aloverose and the (partial) acetylated version of Glucomannan are compounds that occur in *Aloe vera* plants. In the present invention, it has surprisingly been discovered that a combination of rhubarb *(Rheum palmatum)* root extract and at least one further compound, selected from the group consisting of Acemannan and/or Glucomannan leads to a synergistic antibacterial effect resulting in a significant reduction of periodontal bacteria such as *Porphyromonas gingivalis* without harming commensal bacteria such as *Streptococcus oralis.* Furthermore, the combination of *Rheum palmatum* root extract and Acemannan and/or Glucomannan synergistically enhances wound healing properties. Acemannan and/or Glucomannan are compounds that can be found, for instance, in *Aloe vera* plants. These properties make the inventive combination suitable in the prevention or treatment of oral or dental disorders or dysfunctions, and for the preparation of mouth rinse solutions.

As shown by the present invention, the combination of *Rheum palmatum* root extract in addition to Acemannan and/or Glucomannan, or plant extracts containing said compounds is more efficient than *Rheum palmatum* root extract or *Aloe vera* extract alone.

The synergistic effect has been determined by calculation of the Fractional Inhibitory Concentration Index (FICI), which is used to determine the interaction of the two drugs in combination (Tamura T, Asahara M, Yamamoto M, et al. In vitro susceptibility of dermatomycoses agents to six antifungal drugs and evaluation by fractional inhibitory concentration index of combined effects of amorolfine and itraconazole in dermatophytes. Microbiol Immunol. 2014;58(1):1-8; Odds FC. Synergy, antagonism, and what the chequerboard puts between them. J Antimicrob Chemother. 2003;52(1):1). As such, the FICI represents the sum of the Fractional Inhibitory Concentrations (FICs) of each substance tested, where the FIC is determined for each substance by dividing the Minimum Inhibitory Concentrations (MIC) of each substance when used in combination by the MIC of each substance when used alone. As shown in the present invention, the Fractional Inhibitory Concentration Index is $\leq 0.5$ for the combination of *R. palmatum* and *Aloe vera* plant extract.

Preferably the compositions of the present invention are formulated as a mouth rinse or dentifrice formulation for the prevention and treatment of oral disorders or diseases. Such disorders or diseases include, but not limited to dental caries, gingivitis, periodontitis or peri-implantitis, sores, cuts, periodontopathic bacteria, microbial biofilm formation, and/or plaque formation. Due to their wound healing and antimicrobial properties, the compositions according to the present invention can also be used as mouth rinse solutions for use in oral or dental care. As a further aspect, a dental disorder that can be treated with the compositions, and in particular the mouth rinse solutions of the present invention, is dental caries.

In a preferred embodiment, the composition of the present invention contains rhubarb root extract and Acemannan, which refers to D-isomer mucopolysaccharide and can be found in *Aloe vera* leaves. In a further preferred embodiment, the composition of the present invention contains rhubarb root extract and Glucomannan as further additive. Glucomannan is a water-soluble polysaccharide that can be used in combination with Acemannan as a preferred additive in the compositions of the present invention. In a preferred embodiment, said Acemannan and/or Glucomannan is obtained from ginseng (*Panax ginseng*), *Aloe vera,* taiga root, and/or devil's tongue (*Amorphophallus konjac*). The additives of the present invention can also be used in arbitrary combination. A preferred composition therefore contains rhubarb root extract, Acemannan, and Glucomannan.

As an alternative to the addition of one or more single components as additive, Acemannan and/or Glucomannn can be replaced by whole plant extracts or extracts derived from plants or plant parts. In a preferred embodiment, the additive relates to a plant extract selected from the group consisting of *Aloe vera* plant extract, or *Orchis spp.* (Orchid) extract. The plant extract to be combined with rhubarb root extract to achieve the desired synergistic effect is preferably obtained from *Aloe vera* leaves or *Orchis spp.* tuber extract.

In a preferred embodiment, a combination of *Rheum palmatum* root extract and *Aloe vera* leave extract is provided. Preferably, the amount of *Rheum palmatum* root extract utilized in the composition of the present invention ranges between about 2 mg/L and about 4 mg/L. Preferably, the amount of *Aloe vera* plant extract as utilized in the composition of the present invention is about 250 μg/mL to about 2000 μg/mL, preferably between 400 and 600 μg/mL, most preferably around 500 μg/mL.

In a preferred embodiment, the composition of the present invention comprises one or more additional components, which are preferably selected from the group consisting of Bromelain or other enzymatic whitening agents and antioxidants, menthol, thymol, eucalyptol, cocamidopropyl betaine or other surfactants, xylitol or other humectants, and/or one or more fluoride ion source.

In a preferred embodiment, the composition comprises 1 wt% bromelain as enzymatic whitening agent and antioxidant, 0.04 wt% menthol, 0.06 wt% thymol and/or 0.09 wt% eucalyptol.

In a preferred embodiment, said fluoride ion source is selected from the group consisting of sodium fluoride, potassium fluoride, sodium monofluorophosphate, sodium fluorosilicate, ammonium fluorosilicate, amine fluoride, ammonium fluoride, and combinations thereof.

Preferably, the fluoride in the compositions of the present invention is provided in form of sodium fluoride and/or as amine fluoride, more preferably as a mixture of sodium fluoride and amine fluoride such that the amount ratio fluoride ions derived from sodium fluoride:fluoride ions derived from amine fluoride is in the range of 0.7:1 to 1.4: 1, more preferably in the range of 0.9:1 to 1.1:1.

In preferred embodiments, the compositions of the present invention are prepared to be used as dentifrice compositions or oral care mouth rinse solutions. Preferably, the additional component fluoride ion source is used in an amount corresponding to > 1000 ppm fluoride, preferably around 1450 ppm fluoride based on the total weight of the composition in a dentifrice composition. When the composition is used as oral care mouth rinse solution, the fluoride ion source is preferably used in an amount corresponding to < 500 ppm fluoride, preferably around 220 ppm fluoride based on the total weight of the composition.

In a preferred embodiment the mouth rinse solution according to the present invention comprises a suitable solvent, carrier, or vehicle. One example for such a solvent includes, but is not limited to polyol solvent, preferably selected from the group consisting of polyhydric alkanes, polyhydric alkane esters, polyalkene glycols, and mixtures thereof. A further possible solvent for the mouth rinse solution is ethanol, in amounts of typically varying between 5% and 15%, based on the mouth rinse. The mouth rinse solution may have a pH which is physiologically acceptable, and which preferably serves to fully dissolve the *R. palmatum* root extract and the one or more additives that lead to the desired synergistic effect. Such pH may typically be in the range of about 3.0 to about 6.0, preferably about 4.0 to about 5.5, more preferably about 4.3 to about 5.0. If necessary, the pH of the mouth rinse solution may be adjusted to the desired value by adding acid (such as hydrochloric acid) or base (such as sodium hydroxide). The mouth rinse solution may also contain flavorings, cooling flavors, or sweeteners. In a preferred embodiment, the mouth rinse solution is formulated as oil-in-water emulsion.

The dentifrice composition is preferably provided as a paste, gel, or as liquid formulation, and may contain an orally acceptable carrier or vehicle. The orally acceptable carrier or vehicle may optionally include at least one phosphate compound. The dentifrice composition according to the present invention may be provided in any desired form, such as deep striped, surface striped, multilayered, or any combination thereof.

The composition of the present invention may also be formulated as a pharmaceutical composition, comprising at least one pharmaceutically acceptable carrier, orally acceptable carrier, or excipient. Such carriers or excipients, include, but are not limited to conventional additives, in particular those used in oral care compositions including, but not limited to fluoride ion sources, antimicrobial agents, anti-inflammatory agents, anti-calculus or anti-tartar agents, desensitizing agents, peroxide sources, abrasives such as silica, buffering agents, alkali metal bicarbonate salts, thickening materials, humectants, water, surfactants, emulsifying agents, antistain agents, tooth substantive agents, titanium dioxide, xylitol, essential oils, a coolant, a sweetening agents, or coloring agents. Preferred orally acceptable carriers include, but are not limited to usual components of toothpastes, tooth powders, prophylaxis pastes, mouth rinses, lozenges, gums, and the like.

Brief description of the Figures

**Figure 1** shows the effect of *R. palmatum* root extract on planktonic S. *oralis* and *P. gingivalis.*

**Figure 2** shows the effect of the combination of *R. palmatum* root extract and *Aloe vera* on cellular migration of fibroblasts.

**Figure 3** shows the effect of *R. palmatum* root extract on cellular migration of fibroblasts.

**Figure 4** shows the effect of *Aloe vera* on cellular migration of fibroblasts.

**Figure 5** shows the effect of different concentrations of *Aloe vera, R. palmatum root extract* and their combination on cellular migration of fibroblasts.

**Figure 6** shows the result of an HPLC analysis using *R. palmatum* root extract and the anthraquinone rhein.

**Figure 7** shows the HPLC chromatogram of the *Aloe vera* extract.

**Figure 8** shows a quantitative analysis of the components of the *Aloe vera* extract as analysed by HPLC.

## Modes for carrying out the invention:

The present invention is illustrated in more detail in the following examples. By no means shall the present invention be limited to those examples.

## Examples

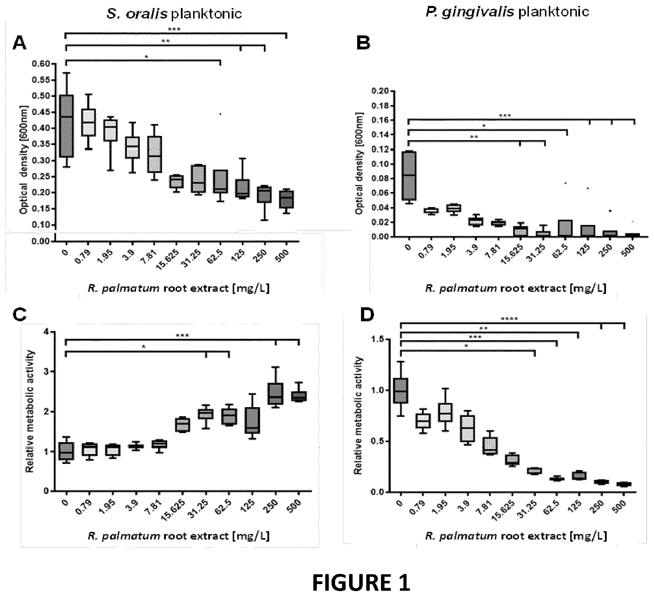

Figure 1 shows, as a reference example, the effect of *R. palmatum* root extract on planktonic S. *oralis* and *P. gingivalis.* Planktonic growth of *P. gingivalis* was significantly inhibited by lower concentrations of *R. palmatum* root extract than growth of S. *oralis.* (A) Treatment with 62.5 mg/L *R. palmatum* root extract and above resulted in significant reduction of the optical density of the bacterial growth of S. *oralis.* (B) Treatment with 15.625 mg/L *R. palmatum* root extract and above resulted in significant reduction of the optical density of *P. gingivalis.* (C) Treatment with 31.25 mg/L *R. palmatum* root extract and above resulted in significant increase of the relative metabolic activity of S. *oralis.* (D) Treatment with 31.25 mg/L *R. palmatum* root extract and above resulted in significant decrease of the relative metabolic activity of *P. gingivalis.* Kruskal-Wallis test with Dunn's correction for multiple comparisons, N = 6 with S. *oralis* strain ATCC 9811 and *P. gingivalis* strain ATCC 33277 / DSM 20709. *$p < 0.05$, ***$p < 0.001$, ****$p < 0.0001$.

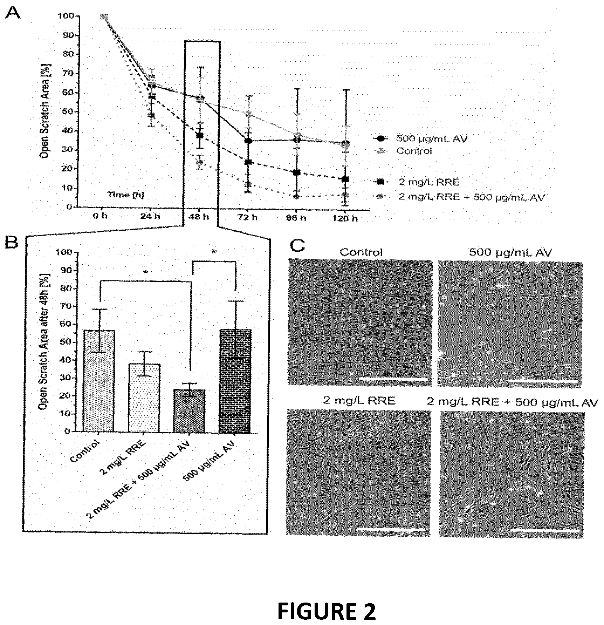

Figure 2 shows the effect of a combination of *R. palmatum* root extract (RRE) and *Aloe vera* (AV) according to the present invention on cellular migration of fibroblasts. The cellular migration of fibroblasts is one necessary part for wound healing. The scratch assay showed that the combination of *R. palmatum* root extract and *Aloe vera* significantly increased the migration of human primary fibroblasts. (A) Treatment with 2 mg/L *R. palmatum* root extract or a combination of 2 mg/L *R. palmatum* root extract and 500 μg/mL *Aloe vera* resulted in nearly complete closure of the scratch area within 96 - 120 h. The combination of 2 mg/L *R. palmatum* root extract and 500 μg/mL *Aloe vera* resulted in fastest gap closure. In this treatment group the gap was closed more than 90% already after 96 h. In contrast, the scratch area in the control group was still 35 % open at that point. (B) After 48 h, the open scratch area of the control group remained > 50 % open. The combination of 2 mg/L *R. palmatum* root extract and 500 μg/mL reduced the open scratch area to < 25%, whereas the gap remained > 40% open if 500 μg/mL *Aloe vera* was used alone. Thus, the combination of both substances significantly improved gap closure compared to *Aloe vera* alone and compared to the untreated control group. One-way ANOVA, comparison between all groups, N = 3 with human primary fibroblasts from different patients, * $p < 0.05$. (C) Representative images of the open scratch of the control group and 2 mg/L *R. palmatum* root extract per 500 μg / mL *Aloe vera* after 48 h. Scale = 200 μm.

Taken together, a combination of *R. palmatum* root extract and *Aloe vera* increased the migration of human primary fibroblast much more effectively than *R. palmatum* root extract or *Aloe vera* alone.

More specifically, a combination of *R. palmatum* root extract and *Aloe vera* surprisingly resulted in synergistic effects regarding wound healing *in vitro.*

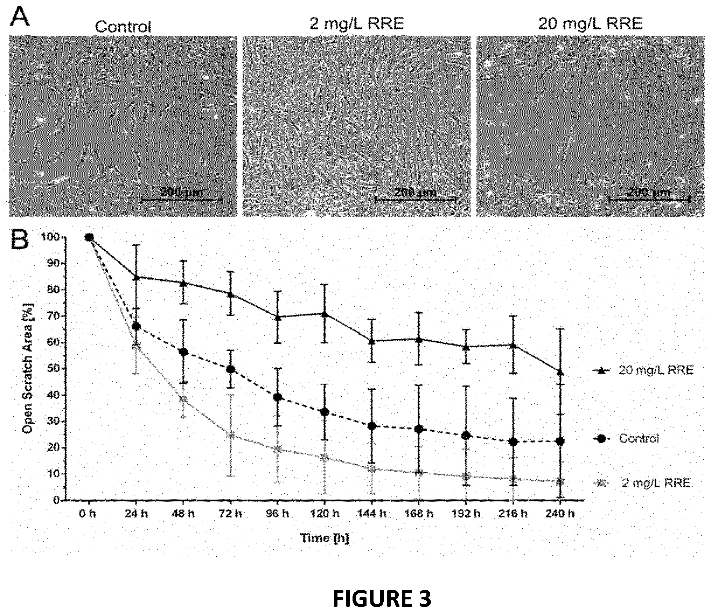

Figure 3 shows, as a reference example, the effect of *R. palmatum* root extract (RRE) on cellular migration of fibroblasts. The scratch assay showed that 2 mg/L of *R. palmatum* root extract increased the migration of human primary fibroblasts, whereas 20 mg/L *R. palmatum* root extract decreased fibroblast migration. (A) Representative images of the open scratch area in the different treatment groups after 72 h. Treatment with 2 mg/L *R. palmatum* root extract resulted in nearly complete closure of the scratch area within 144-240 h, whereas gap closure did not occur even after 240 h with 20 mg/L RRE. (B) After 72 h, the scratch area of the control group remained 50 % open. Low concentration of 2 mg/L of *R. palmatum* root extract reduced the open scratch area to 25%. With high concentrations of 20 mg/L *R. palmatum* root extract the scratch area remained >75% open after 72 h. N = 3 with human primary fibroblasts from different patients. Taken together, the efficiency of the *R. palmatum* root extract was significantly lower than the inventive combination of *R. palmatum* root extract and *Aloe vera.*

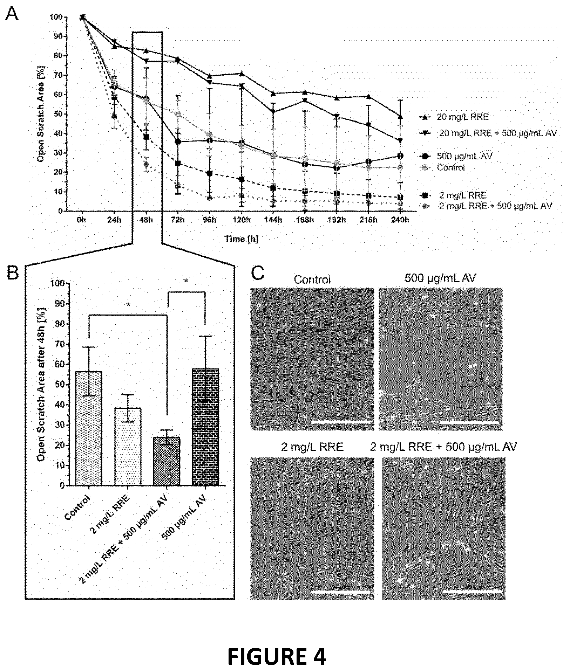

Figure 4 shows, as a reference example, the effect of *Aloe vera* (AV) on cellular migration of fibroblasts. The scratch assay showed that concentrations of *Aloe vera* above 100 μg/mL significantly increased the migration of primary human fibroblasts. (A) After 240 h the scratch area was still 50 % open in the control group, while after treatment with 500 mg/mL, 1.000 μg/mL, or 2.000 μg / mL *Aloe vera* the gap was completely closed. (B) After 144 h, the scratch area of the control group was still open for 70% whereas high concentrations of *Aloe vera* above 500 μg/mL resulted in gap closure of 35%. Two-way ANOVA, comparison of each treatment group with untreated control group, N = 3 with human primary fibroblasts from different patients, * $p < 0.05$, *** $p < 0.001$ (C) Representative images of the gap closure in the different treatment groups after 144 h. Taken together, the efficiency of the *Aloe vera* was significantly lower than the inventive combination of *R. palmatum* root extract and *Aloe vera.*

From the definition of pCI_AV and pCI_RRE; the curves (Figure 4A) are strictly monotonic decreasing and do not intersect. Because RRE+AV show much lower values for all times, both pCI-ratios, pCI_AV and pCI_RRE, will be strict <1 by elementary analysis. Calculation at t=168 h yields pCI_AV=5.34/24.23=0.22 and pCI_RRE=5.34/10.53=0.51. According to the experimental setup, quotients < 1 are sufficient to assume synergy.

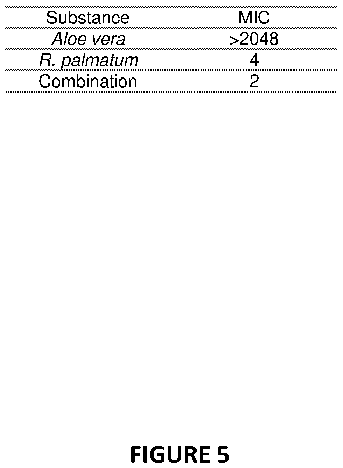

Figure 5 shows the effect of different concentrations of *Aloe vera, R. palmatum root extract* and their combination on cellular migration of fibroblasts. The broth microdilution assay revealed synergistic antibacterial effects if *R. palmatum* root extract and *Aloe vera* are combined. *Aloe vera* alone showed a Minimum Inhibitory Concentration (MIC) of > 1024. *R. palmatum* root extract alone showed a MIC of 4 mg/L which would be suspected for the combination of *Aloe vera* and *R. palmatum* root extract. The MIC of *R. palmatum* root extract combined with *Aloe vera* was 2mg /L indicating a synergistic effect as calculated by Fractional Inhibitory Concentration Index (FICI). The combination of *Aloe vera* and *R. palmatum* root extract showed a FICI of 0.48 against P. gingivalis defined as synergy (FICI ≤0.5).

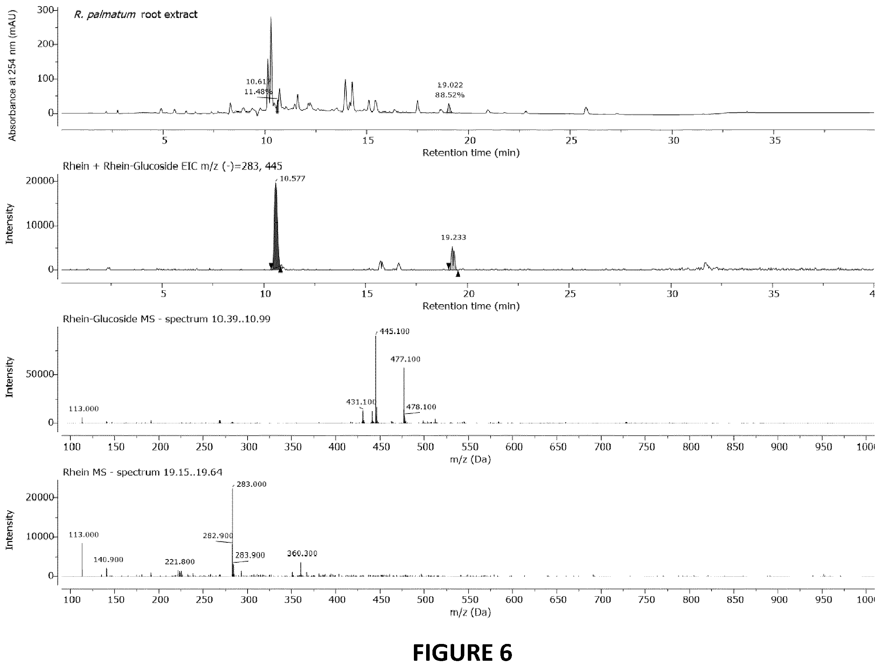

Figure 6 shows the result of an HPLC analysis of *R. palmatum* root extract and the anthraquinone rhein. The HPLC chromatogram was monitored at 254 nm. Respective total ion current (TIC) ESI-MS (-) and extracted ESI-MS (-) spectrum of *R. palmatum* root sample at 10.39-10.99 min (rhein-glucoside) and 19.21-19.34 min (rhein). The HPLC analysis shows the presence of anthraquinones and a rhein concentration of 5% in the *R. palmatum* root extract By comparing the retention time of rhein with the signals observed for the *R. palmatum* extract, it was noted that rhein was present in the *R. palmatum extract* sample, but in a relatively low concentration. The comparison of the UV signal levels between the rhubarb root and the pure rhein sample indicated a rhein concentration of 5% in the *R. palmatum* extract. An extracted ion chromatogram (EIC) of rhein glucoside (m/z [M - H]- = 445.1) and rhein (m/z [M - H]- = 283.0) confirmed the presence of both forms. The m/z values of other known R. *palmatum* anthraquinones were also detected but could not be individually assigned.

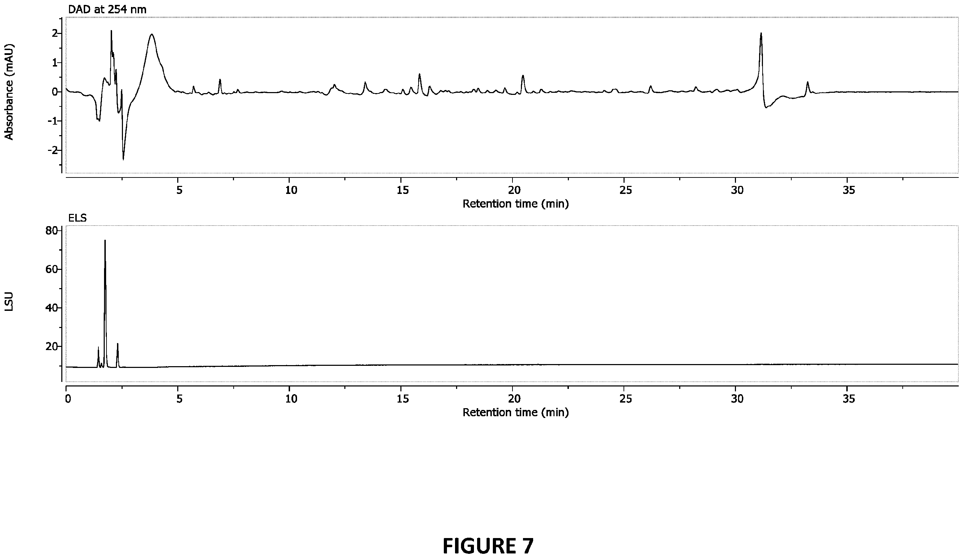

Figure 7 shows the HPLC chromatogram of the *Aloe vera* extract. Shown are the traces of a diode array detector (DAD) monitored at 254 nm and an electrophoretic light scattering (ELS) detector. The HPLC analysis showed that the AV sample consisted almost exclusively of water-soluble aliphatic compounds. NMR analysis revealed the main components to be acemannan, glucose, malic acid, and citric acid.

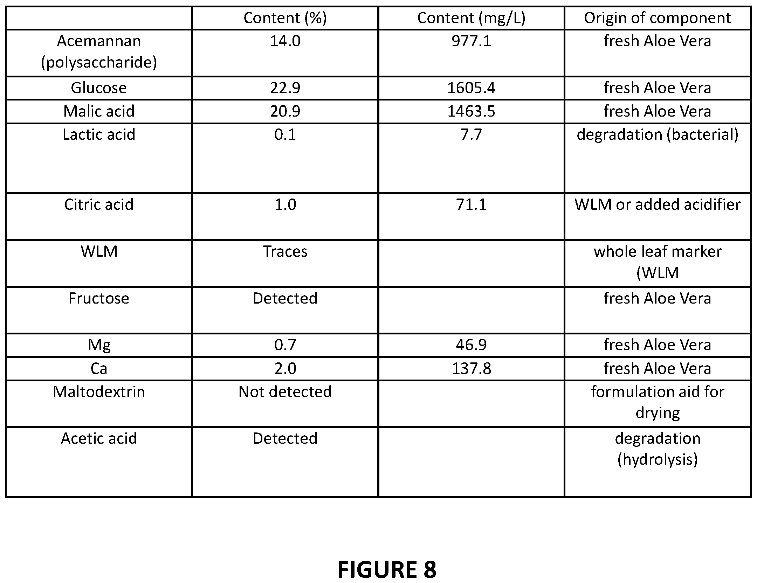

Figure 8 shows a quantitative analysis of the components of the *Aloe vera* extract as analysed by HPLC. The main component is Acemannan (14%). Aloe Emodin was not detectable in the sample.

The results of Figures 6, 7 and 8 show that not only extracts of *R. palmatum* root extract or *Aloe vera* can be used but any composition comprising Acemannan and/or Glucomannan.

In summary, the efficiency of the inventive combination of *R. palmatum* root extract and *Aloe vera* is significantly higher than of *R. palmatum* root extract or *Aloe vera* alone.

## Material and Methods

### *Cell Isolation and Cell Culture*

Primary human fibroblasts were isolated from mucosa obtained from patients who underwent surgery at the Department of Otorhinolaryngology, University Medical Center Mainz, Germany. This study was performed in agreement with the declaration of Helsinki on the use of human material for research. In accordance with the ethics committee of Rhineland-Palatinate, patients agreed with the scientific use of the surplus material and no further approval of the medical ethics committee was required as the fibroblasts were used anonymously. Tissue samples were cut into small pieces of approximately $2 \times 2$ mm with a sterile disposable scalpel. Prior to cell isolation, the tissue pieces were stepwise sterilized in 70% ethanol, in sterillium® classic pure (Bode Chemie GmbH, Hamburg, Germany), and again in 70% ethanol. Then they were transferred to 5-10 mL (depending on the amount of tissue) 0.5% protease solution (P6141, Sigma-Aldrich, St. Louis, MO, US) in phosphate buffered saline (PBS; Sigma-Aldrich, St. Louis, MO, US) and incubated overnight at 4°C. The next day, the protease solution was incubated with shaking for further 15 min at 37°C. The sample was then passed through a cell sieve (EASYstrainer™ 70 μm sterile, Greiner bio-one, Kremsmünster, Austria) with the help of a cell scraper (Falcon®, Corning, NY, US). Cells were pelleted by centrifugation (1500 rpm, 5 min), were transferred to cell culture medium, and seeded into small cell culture flasks with 25 cm² grow area. Cells were characterized morphologically and were used at most until passage 10 to ensure primary identity. Cells were maintained in DMEM/Ham's F12 (Gibco, ThermoFisher Scientific, Waltham, MA, USA) supplemented with bovine calf serum (VWR International GmbH, Hessen, Germany) and antibiotics (10000 U/mL penicillin and 10 mg/mL streptomycin, Sigma-Aldrich, St. Louis, MO, USA) at 37°C in 5% $CO_2$.

### *Microorganisms and Culture Conditions*

*P. gingivalis* (DSM No. 20709, ATCC 33277) was obtained from the German Collection of Microorganisms and Cell Cultures (DSMZ) and cultivated on Schaedler Agar (Becton-Dickinson, Heidelberg, Germany) to obtain 48-hour-old cultures for further processing. A working aliquot was held at -70°C and used for weekly sub-culturing. Anaerobic cultivation was performed under anaerobic conditions (90% $N_2$, 10% $CO_2$, 10% $H_2$) in an anaerobic jar system (Anoxomat Mart II, Mart Microbiology BV, Lichtenvoorde, Netherlands).

*Broth Microdilution Assay*

MIC was determined by serial microdilution according to the Clinical and Laboratory Standards Institute (CLSI) guidelines. Bacterial suspensions of $1-5 \times 10^7$ colony-forming units (0.5 McFarland standard) were used. Of these suspensions, 1 mL was added to 9 mL Wilkins Chalgrens broth (Merlin GmbH, Bornheim-Hersel, Germany). Microdilution was performed using sterile 96-well plates (Greiner Bio-One GmbH, Frickenhausen, Germany) with a final test volume of 100 $\mu$L per well. Column one served as negative control (sterile broth only) and column two served as positive control (bacterial suspension only). A serial dilution of each test substance was performed starting in column 4 as quadruples. A solution with either 2048 mg/L AV or 2048 mg/L RRE or 1024 mg/L RRE and 1024 mg/L AV was used as start concentration in column 4. After the potentially antimicrobial agent has been diluted, the same volume of the standardized bacterial suspension was added to each well (50 $\mu$L). A solvent control (50% distilled water/50% ethanol) was performed in the same way on a separate plate to exclude antibacterial activity of the solvents. After an incubation period of 48 h for *P. gingivalis* at 37°C, the plates were inspected. The MIC was determined as the lowest concentration where no visible growth was seen in the wells. Each test was repeated three times.

*Preparation of RRE/AV Solutions*

RRE and AV were obtained from Paninkret (Paninkret, Pinneberg, Germany). The substances were dissolved in sterile filtered, deionised, and autoclaved water and the solutions were adjusted to concentrations of 10-2048 mg/L. Solutions were freshly prepared for each experiment. The identity of the RRE extract used in this study was identified according to the European Pharmacopeia and the European Union herbal monograph on *Rheum palmatum L.* and *Rheum officinale Baillon,* radix (EMEA/HMPC/189624/2007). AV was obtained from *Aloe barbadensis* Miller and its identity was identified according to the European Union herbal monograph on *Aloe barbadensis Mill.* and on *Aloe* (various species, mainly *Aloe ferox* Mill. and its hybrids), folii succus siccatus.

*HPLC (high-performance liquid chromatography) analysis*

Analysis of AV was performed on an Agilent Infinity II 1260 system with a diode array detector. An ACE C18-PFP column (150 mm $\times$ 4.6 mm, 3 $\mu$m, 40°C) was applied as stationary phase. A gradient mixture of acetonitrile and water (containing 0.1% formic acid) with a constant flow rate of 1.0 mL/min was used as an eluent with the following linear gradient elution program: 0 min: 99% $H_2O$ and 1% MeCN, 30 min: 5% $H_2O$ and 95% MeCN, 40 min: 5% $H_2O$ and 95% MeCN. For HPLC analysis, the material was dissolved in a 1:1 (v/v) MeCN/$H_2O$ solution arriving at a concentration of 1.02 mg/mL. The resulting sample was filtered over a Macherey-Nagel syringe filter with a PTFE membrane (0.2 $\mu$m pore size) prior to injection with an injection volume of 3 $\mu$L.

The HPLC analysis of RRE (Paninkret, Pinneberg, Germany) has been published in a previous study [32].

*NMR (nuclear magnetic resonance)*

NMR spectra were recorded on a Bruker (Billerica, MA, USA) Avance-III (1H-NMR: 600 MHz, 13C-NMR: 151.1 MHz) spectrometer. Chemical shifts are referenced to the residual solvent signal (D2O: 4.79 ppm for 1H-NMR) and reported in parts per million (ppm) relative to tetramethylsilane (TMS).

*Cellular Viability*

Cells were seeded into a 96-well-plate (10000 cells/well in 250 $\mu$L cell culture medium) and they were given time to adhere overnight. After 24 h, cells were treated with 2 mg/L to 200 mg/L RRE (Paninkret Chem.-Pharm. Vertriebsgesellschaft mbH, Pinneberg, Germany) and/or 10 $\mu$g/mL to 2000 $\mu$g/mL AV extract (Paninkret Chem.-Pharm. Vertriebsgesellschaft mbH). The substances were suspended in cell culture medium without FCS. Untreated cells that were equally maintained in medium without FCS served as control. After 24 h treatment, medium was exchanged for medium with 10% AlamarBlue™ Cell Viability Reagent (ThermoFisher Scientific, Waltham, MA, USA) and the cells were incubated for 4 h at 37°C. The AlamarBlue assay is based on the change of the blue colour of the non-fluorescent indicator dye (resazurin) after acceptance of electrons, which, passing from the oxidized state to the reduced state, becomes a fluorescent pink compound [35]. Fluorescence was measured on a Fluorescence Microplate Reader (Fluoroskan Ascent Microplate reader, ThermoFisher Scientific, Waltham, MA, USA). Results were given as relative fluorescence using a 538 nm excitation filter and a 600 nm emission filter, normalized to untreated control.

*Migration Assay*

To analyse the cellular migratory capacity, culture-inserts in a 35 mm $\mu$-Dish-system (ibidi® GmbH, Munich, Germany) were used. 24 h before treatment, 28000 cells were seeded in each chamber within 70 $\mu$L of cell culture medium and incubated overnight. After removal of the insert the cells were treated with the indicated amount of RRE and/or AV. The substances were suspended in cell culture medium without FCS. Untreated cells that were equally maintained in medium without FCS served as control. The defined scratch in the cell layer was photographed after every 24 h for 10 d (24 h-240 h). For computer-assisted objective evaluation of the gap closure the "T-scratch"-software (www.cse-lab.ethz.ch/software.html) was used.

*Phenomenological Combination Index (pCI)*

To establish and determine notions of synergy and antagonism, a method based on median effect principles was established and derived from generalized mass action considerations by fundamental considerations. Due to the inventor's setup, however, by acting with plant extracts on extended - although well-defined - cell culture samples, the strict principle-based index of Chou et al. (Chou, T.C.; Talalay, P. Quantitative analysis of dose-effect relationships: the combined effects of multiple drugs or enzyme inhibitors. Adv Enzyme Regul 1984, 22, 27-55, doi:10.1016/0065-2571(84)90007-4.) has been modified into a phenomenological index pCI to reflect some differences.
As such, we use the quotients pCI_AV= f_RREAV/f_AV and pCI_RRE= f_RREAV/f_RRE which describe the individual relations of the two extracts AV and RRE. According to the experimental setup, quotients < 1 are sufficient to assume synergies and show the effects of faster closure in comparison of the extracts and thus a better phenomenological efficiency under the above assumptions.

*Determining the Fractional Inhibitory Concentration Index (FICI)*

The Fractional Inhibitory Concentration Index (FICI) was calculated using the following formula:

$$FICI = (MIC_A^{combi}/MIC_A^{alone}) + (MIC_B^{combi}/MIC_B^{alone})$$

"Synergy" was defined according to Odds *et al.* as a $\geq$ 4-fold reduction in the Minimum Inhibitory Concentrations (MICs) of both compounds in combination compared to their MICs alone (FICI $\leq$ 0.5); "no interaction" when the MICsyn of one of the compounds remained in the range of $\frac{1}{2}$ x to 4 x MIC (FICI > 0.5 - 4); and "antagonism" when the MICsyn of both compounds is, at least, 4-fold higher than compared to the activity of the compounds alone (FICI > 4.0) (Odds FC. Synergy, antagonism, and what the chequerboard puts between them. J Antimicrob Chemother. 2003 Jul;52(1):1. doi: 10.1093/jac/dkg301. Epub 2003 Jun 12. PMID: 12805255.).

**Claims**

1. A composition comprising rhubarb *(Rheum palmatum)* root extract and one or more additives selected from the group consisting of Acemannan and/or Glucomannan, or plant extracts containing said compounds for use in the prevention or treatment of an oral or dental disorder or dysfunction.

2. The composition for use according to claim 1, wherein said Acemannan and/or Glucomannan is obtained from ginseng (*Panax ginseng*), *Aloe vera,* taiga root and/or devil's tongue (*Amorphophallus konjac).*

3. The composition for use according to claim 1, wherein said additive is a plant extract selected from the group consisting of *Aloe vera* plant extract or *Orchis spp.* tuber extract.

4. The composition for use according to any one of claims 1 to 3, wherein said composition is formulated as a mouth rinse or dentifrice formulation.

5. The composition for use according to any one of claims 1 to 4, wherein said oral disorder is selected from gingivitis, periodontitis or peri-implantitis, sores, cuts, periodontopathic bacteria, microbial biofilm formation and/or plaque formation.

6. The composition for use according to any one of claims 1 to 5, wherein said dental disorder is dental caries.

7. The composition for use according to any one of claims 1 to 6, wherein said *Rheum palmatum* root extract is present in the composition in an amount of from about 2 mg/L to 4 mg/L.

8. The composition for use according to claim 3, wherein said *Aloe vera* plant extract is present in said composition in an amount of from about 500 $\mu$g/mL to about 2000 $\mu$g/mL.

9. The composition for use according to any one of claims 1 to 8, comprising one or more additional components selected from the group consisting of

   a) Bromelain or other enzymatic whitening agent and antioxidant,
   b) menthol,
   c) thymol,
   d) eucalyptol,
   e) cocamidopropyl betaine or other surfactants,
   f) xylitol or other humectants,
   g) fluoride ion source.

10. The composition for use according to claim 9, wherein said fluoride ion source is selected from the group consisting of sodium fluoride, potassium fluoride, sodium monofluorophosphate, sodium fluorosilicate, ammonium fluorosilicate, amine fluoride, ammonium fluoride, and combinations thereof.

11. The composition for use according to any one of claims 9 to 10, wherein said fluoride is present in said composition in a ratio of 220 ppm to 1450 ppm based on the total weight of the composition.

12. A mouth rinse solution or dentifrice composition for use in oral or dental care, comprising a composition according to any one of claims 1 to 11, and a suitable solvent, carrier, or vehicle.

FIGURE 1

**FIGURE 2**

FIGURE 3

EP 4 324 471 A1

**FIGURE 4**

| Substance | MIC |
|---|---|
| *Aloe vera* | >2048 |
| *R. palmatum* | 4 |
| Combination | 2 |

**FIGURE 5**

EP 4 324 471 A1

**FIGURE 6**

FIGURE 7

| | Content (%) | Content (mg/L) | Origin of component |
|---|---|---|---|
| Acemannan (polysaccharide) | 14.0 | 977.1 | fresh Aloe Vera |
| Glucose | 22.9 | 1605.4 | fresh Aloe Vera |
| Malic acid | 20.9 | 1463.5 | fresh Aloe Vera |
| Lactic acid | 0.1 | 7.7 | degradation (bacterial) |
| Citric acid | 1.0 | 71.1 | WLM or added acidifier |
| WLM | Traces | | whole leaf marker (WLM |
| Fructose | Detected | | fresh Aloe Vera |
| Mg | 0.7 | 46.9 | fresh Aloe Vera |
| Ca | 2.0 | 137.8 | fresh Aloe Vera |
| Maltodextrin | Not detected | | formulation aid for drying |
| Acetic acid | Detected | | degradation (hydrolysis) |

**FIGURE 8**

Europäisches
Patentamt

European
Patent Office

Office européen
des brevets

## EUROPEAN SEARCH REPORT

Application Number

EP 22 19 0349

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 101 104 548 B1 (HAN SUNG HEE [KR]) 11 January 2012 (2012-01-11) * page 4, paragraph 39; tables 1, 2 * ----- | 1-11 | INV. A61K36/708 A61K36/886 A61K36/258 A61Q11/00 A61P1/02 |
| X | DATABASE GNPD [Online] MINTEL; 13 July 2021 (2021-07-13), anonymous: "Detox Capsules", XP093012149, Database accession no. 8856657 * page 2 * ----- | 12 | |
| X | DATABASE GNPD [Online] MINTEL; 4 April 2017 (2017-04-04), anonymous: "Mouth Ulcer Solution", XP093011825, Database accession no. 4729005 * page 2 * ----- | 1-11 | |
| A,D | Ashouri Moghaddam Anahita ET AL: "Clinical Evaluation of Effects of Local Application of Aloe vera Gel as an Adjunct to Scaling and Root Planning in Patients with Chronic Periodontitis", Journal of Dentistry, 1 September 2017 (2017-09-01), pages 165-172, XP93012277, Iran Retrieved from the Internet: URL:https://www.ncbi.nlm.nih.gov/pmc/articles/PMC5634355/pdf/JDS-18-165.pdf [retrieved on 2023-01-09] * abstract * ----- -/-- | 1-12 | TECHNICAL FIELDS SEARCHED (IPC) A61P A61K A61Q |

The present search report has been drawn up for all claims

| Place of search | Date of completion of the search | Examiner |
|---|---|---|
| Munich | 23 January 2023 | Ramos Barbosa, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 0349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MÜLLER-HEUPT LENA KATHARINA ET AL: "Extracts of Rheum palmatum and Aloe vera Show Beneficial Properties for the Synergistic Improvement of Oral Wound Healing", PHARMACEUTICS, [Online] vol. 14, no. 10, 27 September 2022 (2022-09-27), page 2060, XP093011757, DOI: 10.3390/pharmaceutics14102060 [retrieved on 2023-01-05] * the whole document * | 1-12 | |
| A,D | "Clinical evaluation of effects of local application of Aloe vera gel as an adjunct to scaling and root planing in patients with chronic periodontitis", J DENT SHIRAZ UNIV MED SCI,, vol. 18, no. 3, 1 September 2017 (2017-09-01), pages 165-172, XP002808354, * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 23 January 2023 | Ramos Barbosa, I |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 19 0349

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2023

| Patent document cited in search report | Publication date | Patent family member(s) | Publication date |
|---|---|---|---|
| KR 101104548 B1 | 11-01-2012 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 1263484 C
- CN 107595953 A
- CN 101491614 A
- JP S5758612 A
- CN 103479817 A
- CN 108420933 A

### Non-patent literature cited in the description

- What we already know about rhubarb: a comprehensive review. *Chinese Medicine,* 2020, vol. 15, 88
- *The curative effect of rhubarb extract on severe periodontitis in patients with diabetes mellitus,* December 2018, vol. 27 (6), 633-636
- Rheum emodi L. (rhubarb) promotes wound healing by decreasing inflammatory markers and enhancing accumulation of biomolecules. *Annals of Phytomedicine,* 2017, vol. 6 (2), 114-118
- Antibacterial Effect of Aloe vera Gel against Oral Pathogens: An In-vitro Study. *Journal of Clinical and Diagnostic Research,* 2016, vol. 10 (11), ZC41-ZC44
- The Effect of Aloe vera Clinical Trials on Prevention and Healing of Skin Wound: A Systematic Review. *IJMS,* 2019, vol. 44 (1
- Clinical evaluation of effects of local application of Aloe vera gel as an adjunct to scaling and root planing in patients with chronic periodontitis. *J Dent Shiraz Univ Med Sci,* September 2017, vol. 18 (3), 165-172
- **TAMURA T ; ASAHARA M ; YAMAMOTO M et al.** In vitro susceptibility of dermatomycoses agents to six antifungal drugs and evaluation by fractional inhibitory concentration index of combined effects of amorolfine and itraconazole in dermatophytes. *Microbiol Immunol.,* 2014, vol. 58 (1), 1-8
- **ODDS FC.** Synergy, antagonism, and what the chequerboard puts between them. *J Antimicrob Chemother.,* 2003, vol. 52 (1), 1
- **CHOU, T.C. ; TALALAY, P.** Quantitative analysis of dose-effect relationships: the combined effects of multiple drugs or enzyme inhibitors. *Adv Enzyme Regul,* 1984, vol. 22, 27-55
- **ODDS FC.** Synergy, antagonism, and what the chequerboard puts between them. *J Antimicrob Chemother.,* 12 June 2003, vol. 52 (1), 1